(19) **Europäisches Patentamt / European Patent Office / Office européen des brevets**

(11) **EP 2 793 700 B1**

(12) **FASCICULE DE BREVET EUROPEEN**

(45) Date de publication et mention de la délivrance du brevet:
**03.05.2017 Bulletin 2017/18**

(51) Int Cl.:
***A61B 5/11*** *(2006.01)*     ***A63B 24/00*** *(2006.01)*
***G06K 9/00*** *(2006.01)*

(21) Numéro de dépôt: **12805443.4**

(22) Date de dépôt: **14.12.2012**

(86) Numéro de dépôt international:
**PCT/EP2012/075503**

(87) Numéro de publication internationale:
**WO 2013/092405 (27.06.2013 Gazette 2013/26)**

(54) **SYSTÈME ET PROCÉDÉ DE DÉTECTION D'AU MOINS UNE PHASE TRANSITOIRE DANS UNE ACTIVITÉ STATIONNAIRE D'UN ÊTRE ANIMÉ**

SYSTEM UND VERFAHREN ZUM ERFASSEN VON MINDESTENS EINER ÜBERGANGSPHASE IN EINER KONSTANTEN AKTIVITÄT EINES ANIMIERTEN WESENS

SYSTEM AND METHOD FOR DETECTING AT LEAST ONE TRANSIENT PHASE IN A STEADY ACTIVITY OF AN ANIMATED BEING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.12.2011 FR 1161864**

(43) Date de publication de la demande:
**29.10.2014 Bulletin 2014/44**

(73) Titulaires:
• **Commissariat à l'Énergie Atomique et aux Énergies Alternatives**
**75015 Paris (FR)**
• **Movea**
**38000 Grenoble (FR)**

(72) Inventeur: **MAYOUE, Aurélien**
**91450 Soisy Sur Seine (FR)**

(74) Mandataire: **Brunelli, Gérald**
**Marks & Clerk France**
**Immeuble Visium**
**22, avenue Aristide Briand**
**94117 Arcueil Cedex (FR)**

(56) Documents cités:
**EP-A1- 1 870 139     EP-A2- 2 236 987**
**WO-A1-2010/122173     WO-A1-2011/012666**
**US-B1- 6 571 193**

**Description**

L'invention porte sur un système et un procédé de détection d'au moins une phase transitoire dans une activité stationnaire d'un être animé, à partir de signaux représentatifs du mouvement de l'être animé, tel un humain ou un robot mobile.

Une activité stationnaire se définit par des caractéristiques statistiques de l'activité invariantes par translation dans le temps, tels les moments d'ordre k, et plus particulièrement les moments d'ordre 1 (par exemple une moyenne) et les moments d'ordre 2 (par exemple une corrélation)).

En guise d'exemples d'activités stationnaires, nous pouvons citer, de façon non exhaustive, les activités suivantes : course à pied, natation, équitation, musculation...Ces activités présentent en effet des phases stationnaires pendant lesquelles les caractéristiques statistiques restent invariantes dans le temps. Elles peuvent toutefois être entrecoupées de phases dites transitoires pendant lesquelles l'être animé change son activité.

Une phase transitoire correspond à un état durant lequel l'activité stationnaire de l'être animé est perturbée, et durant lequel il y a une modification des propriétés statistiques. Une phase transitoire peut apparaitre sous différentes formes, par exemple sous la forme d'un obstacle à surmonter (des haies à sauter durant un sprint ou des obstacles à sauter durant une séance d'équitation), d'un changement de direction ou de cap de l'être animé (changement de sens lors d'une série de longueurs effectuée par un nageur ou un coureur à pied), ou d'un bref changement d'activité envisagé par l'être animé pour entrecouper son activité stationnaire (série de flexions/extensions durant un footing). Il est ainsi possible de retrouver différentes phases transitoires au sein de la même activité stationnaire. Citons pour exemple une séance de footing en lignes droites entrecoupée de flexions/extensions et marquée par des changements de direction à la fin de chaque ligne droite. Il est intéressant de pouvoir fournir à l'acteur de l'activité des informations sur cette activité, d'une manière automatique et objective, en détectant et en comptant le nombre d'occurrences d'événements dans son activité. Ainsi, la détection et le comptage des phases transitoires dans une activité peuvent constituer une information pertinente pour l'acteur de l'activité.

La demande de brevet américain US 4932045 porte sur un système qui assure le comptage des longueurs mais avec une intervention manuelle du nageur. Le compteur, fixé sur la main ou au pied du nageur, doit subir un appui contre la paroi de la piscine lors de chaque retournement du nageur afin de s'incrémenter d'une unité. Ce dispositif peut perturber la nage de l'athlète.

La demande de brevet américain US 2007/0293374 A1 divulgue un système permettant de compter automatiquement les longueurs d'un nageur sans l'intervention manuelle de celui-ci. Le compteur d'allers-retours comprend un boitier, des moyens de fixation du boitier sur le nageur, un capteur compas interne au boîtier pour fournir un signal de sortie qui change au moment du retournement du nageur (i.e. en fonction de la direction aller ou retour du nageur dans la piscine) et un processeur programmé pour détecter dans le signal de sortie du capteur le changement de direction du nageur et compter le nombre d'allers-retours du nageur.

Un tel système comporte un certain nombre d'erreurs de détection de retournements.

Il est également connu le document WO 2011/012666 qui divulgue un système de détection d'au moins une phase transitoire dans une activité stationnaire d'un être animé.

Un but défini dans les revendications 1-16 est de proposer un système et un procédé de détection d'au moins une phase transitoire dans une activité stationnaire d'un être animé, permettant notamment d'améliorer les performances de détection du système en diminuant le nombre de fausses détections et de non détections, sans en augmenter le coût de calcul.

Aussi, il est proposé, selon un aspect de l'invention, un système de détection d'au moins une phase transitoire dans une activité stationnaire d'un être animé, comprenant des moyens de détermination de signaux représentatifs du mouvement dudit être animé selon au moins une voie, des moyens de calcul d'un signal résultant représentatif d'un lien statistique entre des échantillons desdits signaux représentatifs dudit mouvement appartenant respectivement à au moins deux fenêtres glissantes sur lesdits échantillons, temporellement décalées, et des moyens de détection d'une phase transitoire à partir dudit signal résultant.

Les moyens de détermination des signaux représentatifs du mouvement, peuvent être constitués de capteurs de mouvement portés par l'être animé, par exemple des capteurs de types accéléromètres, magnétomètres, ou gyromètres. Il est également possible d'utiliser des moyens qui équipent l'environnement dans lequel sont effectués les mouvements. Par exemple des moyens de capture du mouvement optiques, électromagnétiques.

Un tel système permet, à coût réduit de détecter au moins une phase transitoire dans une activité stationnaire d'un être animé.

Selon un mode de réalisation, le système comprend des moyens de filtrage desdits signaux représentatifs dudit mouvement en amont desdits moyens de calcul.

Les moyens de filtrage permettent de sélectionner, au sein desdits signaux représentatifs dudit mouvement, les composantes spectrales d'intérêt et de focaliser le traitement qui suit sur les composantes spectrales majoritairement représentatives de la rupture de stationnarité.

Par exemple, lesdites fenêtres glissantes se recouvrent partiellement.

Ainsi, l'évolution temporelle du lien statistique entre les échantillons issus desdites fenêtres glissantes pourra être calculée plus précisément que lorsque le recouvrement est nul. Ceci permettra au final une détection plus précise des phases transitoires.

En outre, lesdites fenêtres glissantes peuvent être de même taille.

Ainsi, le calcul du lien statistique est simplifié car il est réalisé sans ajout ou suppression d'échantillons au sein desdites fenêtres coulissantes.

Dans un mode de réalisation, les fenêtres glissantes ordonnées temporellement et respectivement indicées de 1 à F, sont temporellement espacées de manière à respecter la relation suivante :

$$T_1 + D_F \leq S$$

dans laquelle :

T$_1$ représente la durée de la première fenêtre indicée 1,
D$_F$ représente le décalage entre la première fenêtre indicée 1 et la dernière fenêtre indicée F, et
S correspond à une durée minimale de l'activité stationnaire entre deux phases transitoires successives.

Ainsi, il existe au moins un instant entre deux phases transitoires successives, pour lequel le lien statistique est calculé à partir d'échantillons provenant tous de la même phase stationnaire. A ces instants, l'amplitude du signal résultant, représentatif du lien statistique, sera minimale et pourra être exploitée pour identifier et séparer les différents types de phases.

Selon un mode de réalisation, lorsque lesdits signaux représentatifs du mouvement sont déterminés selon au moins deux voies de mesure, le système comprend des moyens de fusion des composantes selon chaque voie dudit signal résultant.

Ainsi, il est possible d'augmenter les performances de détection de la phase transitoire, en multipliant les mesures du mouvement et ainsi en augmentant la probabilité que les mesures soient porteuses de la rupture statistique due à la phase transitoire du mouvement. Les moyens de fusion permettent ensuite de se ramener au cas à une composante en fusionnant l'information utile issue de toutes les voies de mesures.

Par exemple, lesdits moyens de fusion peuvent être adaptés pour calculer la somme des composantes selon chaque voie dudit signal résultant.

Il est ainsi possible de condenser l'information à moindre coût.

Dans un mode de réalisation, lesdits moyens de détection sont adaptés pour :

- détecter une première phase transitoire dans l'activité stationnaire lorsque le signal résultant selon une voie unique ou le signal fusionné de sortie des moyens de fusion devient supérieur à un premier seuil ; et
- détecter une autre phase transitoire dans l'activité stationnaire lorsque le signal résultant selon une voie unique ou le signal fusionné de sortie des moyens de fusion, depuis la dernière détection d'une phase transitoire, a été inférieur à un deuxième seuil puis est supérieur audit premier seuil ;

ledit deuxième seuil étant inférieur audit premier seuil.

Il est ainsi possible de détecter une série de phases transitoires dans l'activité stationnaire.

Selon un mode de réalisation, le système comprend des moyens de différenciation adaptés pour effectuer une différence entre deux instants desdits signaux représentatifs du mouvement, et des moyens de détermination de la composante d'énergie maximale des signaux différenciés, lesdits moyens de détection étant adaptés pour détecter une autre phase transitoire dans l'activité stationnaire lorsqu'en outre le signe de ladite composante maximale, lorsque le signal fusionné est supérieur audit premier seuil, est l'opposé du signe de ladite composante maximale lors de la détection précédente.

La précision de détection est ainsi améliorée.

En outre, le système peut comprendre des moyens de comptage du nombre de phases transitoires.

Ainsi, en plus de détecter les phases transitoires, le système peut les compter et aussi fournir l'instant d'occurrence de la phase transitoire dans le temps.

En outre, le système peut comprendre des moyens de chronométrage des phases transitoires et/ou stationnaires.

De nombreux coureurs à pieds désirent connaître le nombre de sprints effectués lors d'une séance de fractionné (série de sprints réalisés sur une distance ou un temps fixe et entrecoupés par de brefs plages de repos sous la forme de trottinements). En général, le coureur se fixe à l'avance un nombre de sprints à atteindre pour valider sa séance d'entrainement. Or, il n'est pas toujours facile d'effectuer cet exercice physique tout en comptant mentalement le nombre de sprints effectués. Le fait de disposer d'un système qui détecte et compte automatiquement les phases transitoires (correspondant en l'espèce les phases de trottinements) qui entrecoupent les sprints permet au coureur d'améliorer les conditions dans lesquelles se déroulent ses entrainements.

Dans un mode de réalisation, lesdits moyens de détection sont adaptés pour détecter des demi-tours d'une personne, entre deux parcours de sens opposés d'une ligne droite, ou pour détecter des changements de direction d'une personne sur un parcours comportant une série de lignes droites.

Selon un mode de réalisation, muni d'un boîtier étanche comprenant lesdits moyens de détermination des signaux représentatifs du mouvement d'un nageur et des moyens de fixation pour lier solidairement ledit boîtier à une partie du corps dudit nageur, ladite activité stationnaire étant la nage d'une longueur de piscine et la phase transitoire étant un demi-tour.

Par exemple, lesdits moyens de comptage sont adaptés pour compter les allers-retours d'un nageur dans une piscine.

En effet, de nombreux nageurs désirent pouvoir évaluer précisément la distance qu'ils ont parcourue lors d'une séance de natation. En général, la longueur effectuée est connue du pratiquant et correspond pour lui à une distance renseignée. Toutefois, le fait de devoir compter le nombre de longueurs ou d'allers-retours est fastidieux, comporte un risque d'erreur non négligeable et pour un nageur de bon niveau peut le perturber et limiter sa performance. Un procédé de comptage des phases transitoires (ici, les retournements du nageur) qui interviennent entre chaque longueur permet de résoudre ces problèmes. En outre, en plus du comptage automatique du nombre de longueurs effectuées, l'invention permet de fournir l'instant de chaque retournement et permet ainsi d'enrichir les informations de comptages fournies au nageur.

Par exemple, ledit calcul du lien statistique comprend une fonction de la covariance.

Selon un autre aspect de l'invention, il est également proposé un procédé de détection d'au moins une phase transitoire dans une activité stationnaire d'un être animé, dans lequel on détermine des signaux représentatifs du mouvement dudit être animé selon au moins un axe, et on calcule un signal résultant représentatif d'un lien statistique entre des échantillons de signaux représentatifs dudit mouvement appartenant respectivement à au moins deux fenêtres glissantes sur lesdits échantillons, temporellement décalées.

L'invention sera mieux comprise à l'étude de quelques modes de réalisation décrits à titre d'exemples nullement limitatifs et illustrés par les dessins annexés sur lesquels :

- les figures 1 à 5 illustrent schématiquement des modes de réalisation d'un système, selon un aspect de l'invention ; et
- les figures 6 à 13 illustrent des signaux correspondant aux traitements mis en oeuvre par le système de la figure 4, selon un aspect de l'invention.

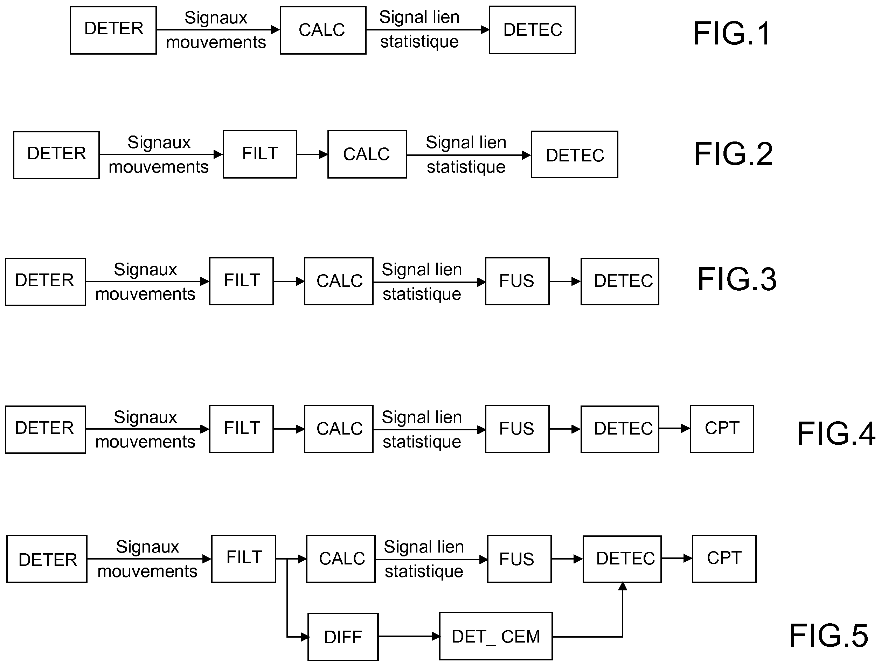

Sur la figure 1, le système de détection d'au moins une phase transitoire dans une activité stationnaire d'un être animé, selon un aspect de l'invention, comprend un module de détermination DETER de signaux représentatifs du mouvement dudit être animé selon au moins un axe, un module de calcul CALC d'un signal résultant représentatif d'un lien statistique entre des échantillons desdits signaux représentatifs dudit mouvement appartenant respectivement à au moins deux fenêtres glissantes, temporellement décalées, et un module de détection DETEC d'une phase transitoire à partir dudit signal résultant.

Un module de filtrage optionnel FILT desdits signaux représentatifs dudit mouvement en amont desdits moyens de calcul, comme représenté sur la figure 2.

Par exemple, les fenêtres glissantes peuvent se recouvrir partiellement, et/ou être de même taille.

En outre, les fenêtres glissantes ordonnées temporellement et respectivement indicées de 1 à F, sont temporellement espacées de manière à respecter la relation suivante :

$$T_1 + D_F \leq S$$

dans laquelle:

$T_1$ représente la durée de la première fenêtre indicée 1,
$D_F$ représente le décalage entre la première fenêtre indicée 1 et la dernière fenêtre indicée F, et
S correspond à une durée minimale de l'activité stationnaire entre deux phases transitoires successives.

En outre, comme illustré sur la figure 3, le système peut comprendre un module de fusion FUS des composantes selon chaque voie dudit signal résultant, par exemple adapté pour calculer la somme des composantes selon chaque voie dudit signal résultant.

En outre, le module de détection DETEC peut être adapté pour :

- détecter une première phase transitoire dans l'activité stationnaire lorsque le signal résultant selon une voie unique ou le signal fusionné de sortie des moyens de fusion FUS devient supérieur à un premier seuil Seuil_1 ; et
- détecter une autre phase transitoire dans l'activité stationnaire lorsque le signal résultant selon une voie unique ou le signal fusionné de sortie des moyens de fusion FUS, depuis la dernière détection d'une phase transitoire, a été inférieur à un deuxième seuil Seuil_2 puis est supérieur audit premier seuil Seuil_1 ;

ledit deuxième seuil Seuil_2 étant inférieur audit premier seuil Seuil_1.

Lesdits seuils Seuil_1 et Seuil_2 dépendent de la plage de mesure du ou des capteurs qui ont servi à acquérir lesdits signaux représentatifs du mouvement, et/ou sont fixés par apprentissage à partir d'une base de signaux de mouvements enregistrés par le ou les capteurs.

En outre, le système peut comprendre un module de comptage CPT du nombre de phases transitoires, comme illustré sur la figure 4.

Le module de détection DETEC peut être adapté pour détecter des demi-tours d'une personne, entre deux traversées de sens opposés d'une ligne droite.

Par exemple, le système peut comprendre un boîtier étanche comprenant les moyens de détermination DETER des signaux représentatifs du mouvement d'un nageur et des éléments de fixations pour lier solidairement le boîtier à une partie du corps du nageur, ladite activité stationnaire étant la nage d'une longueur de piscine et la phase transitoire étant un demi-tour. Le module de comptage CPT peut être adapté pour compter les allers-retours d'un nageur dans une piscine. Aussi, le module de comptage peut en déduire le nombre de longueurs parcourues, donc la distance nagée. Il peut en outre fournir les instants correspondant aux demi-tours détectés. On pourra en déduire une vitesse moyenne de nage, par exemple, sur une ou plusieurs longueurs.

Le calcul du lien statistique peut, par exemple, comprendre une fonction de la covariance.

En outre, comme illustré sur la figure 5, le système peut comprendre un module de différenciation DIFF adapté pour effectuer une différence entre deux instants t et t+∆t (avec ∆t < S) des signaux représentatifs du mouvement, et un module de détermination DET_CEM de la composante d'énergie maximale des signaux différenciés. Le module de détection DETEC est alors adapté pour détecter une autre phase transitoire dans l'activité stationnaire lorsqu'en outre le signe de ladite composante maximale, lorsque le signal fusionné est supérieur au premier seuil Seuil_1, est l'opposé du signe de la composante maximale lors de la détection précédente. L'énergie d'un signal correspond à la somme de ses échantillons au carré. Le nombre de fausse détection est ainsi limité.

L'exemple qui suit est décrit de manière non limitative.

Le module de calcul CALC est adapté pour fournir un signal résultant d'une fonction qui capture la relation statistique entre les parties des signaux issues d'au moins deux fenêtres glissantes temporellement décalées, à partir d'au moins un signal représentatif du mouvement parcouru par les fenêtres glissantes. Le signal résultant se caractérise par des pics au moment des phases transitoires et des passages par des valeurs proches de zéro durant les phases stationnaires. Il est alors possible en fixant des seuils de décision de détecter et compter automatiquement les phases transitoires.

Par exemple, on peut détecter les demi-tours lors d'une série de longueurs effectuée par un nageur ou un sprinteur. Pour l'exemple, les signaux représentatifs du mouvement sont issus d'un magnétomètre à trois axes porté par le sportif. Aussi le module de détermination DETER comprend le magnétomètre à trois axes.

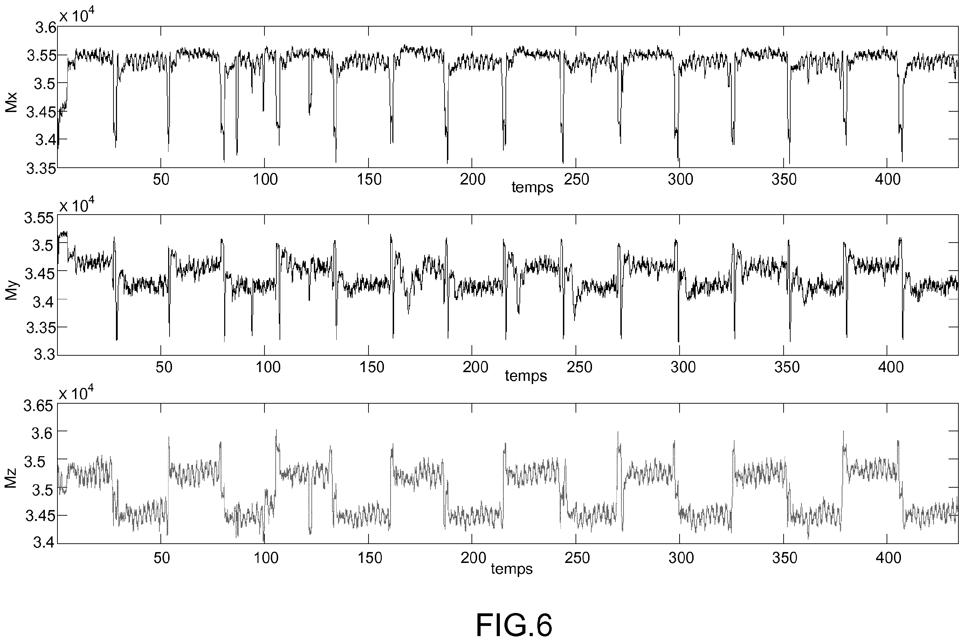

Le magnétomètre à trois axes du module de détermination de DETER fixé sur le corps du nageur ou du sprinteur (tête, dos, cheville ou poignet) fournit un signal échantillonné $M_i(t)$, $i \in \{x, y, z\}$, $t \in \aleph$ qui change en fonction de la direction aller ou retour du sportif. Le signal Mx, My, Mz selon les trois axes x, y et z du magnétomètre est représenté sur la figure 6, correspond à la mesure de seize phases stationnaires entrecoupées de quinze phases transitoires.

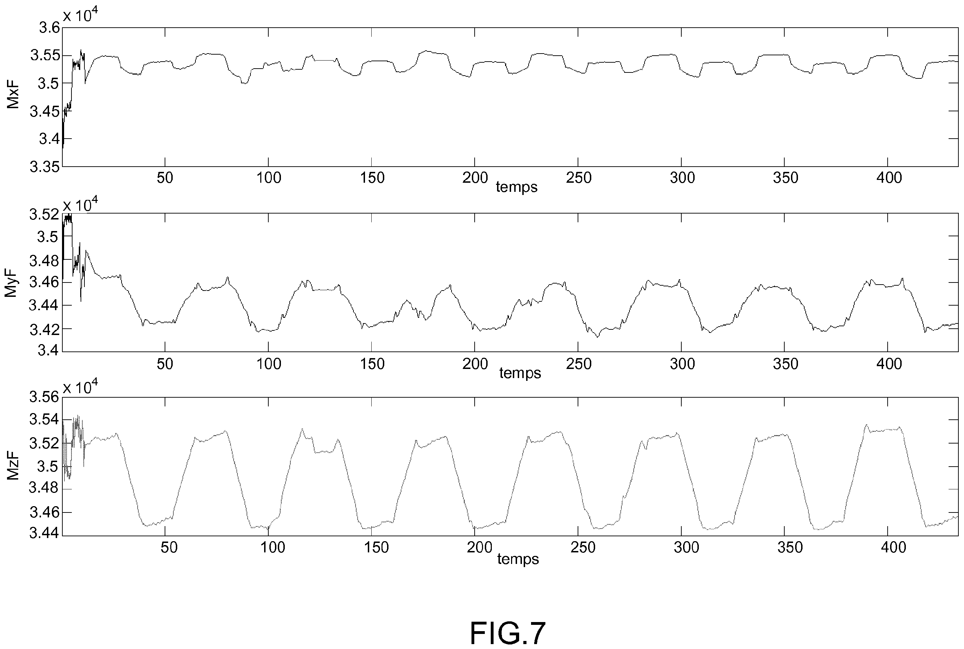

Le module de filtrage FILT permet d'atténuer les mouvements parasites non caractéristiques des phases transitoires et qui peuvent perturber leur détection. Ces mouvements apparaissent durant l'activité stationnaire et sont dus, dans le présent exemple, aux battements du nageur ou aux foulées du sprinteur. Dans ce cas, le filtrage peut être de type passe-bas (filtre moyen) afin de supprimer les mouvements parasites (haute-fréquences) sans affecter l'information utile basse-fréquence liée aux demi-tours comme illustré sur la figure 7 pour chaque axe x, y, z :

$$MF_i(t) = \frac{1}{L} \sum_{n=t-L+1}^{t} M_i(n), \; i \in \{x, y, z\}, \; L \in \aleph$$

La taille $L$ de la fenêtre glissante qui assure le calcul de moyenne doit être telle que $E < L < S$, où $S$ est la durée minimale de l'activité stationnaire entre deux phases transitoires et $E$ représente l'écart maximal entre deux mouvements parasites.

L'étape de filtrage est facultative au sein de la chaîne de traitements, et son application permet toutefois d'améliorer la détection des phases transitoires. Il est possible de substituer à la moyenne d'autres types de filtrage permettant d'atténuer ou de supprimer les composantes du signal correspondant aux mouvements qui ne portent pas d'information de la phase transitoire.

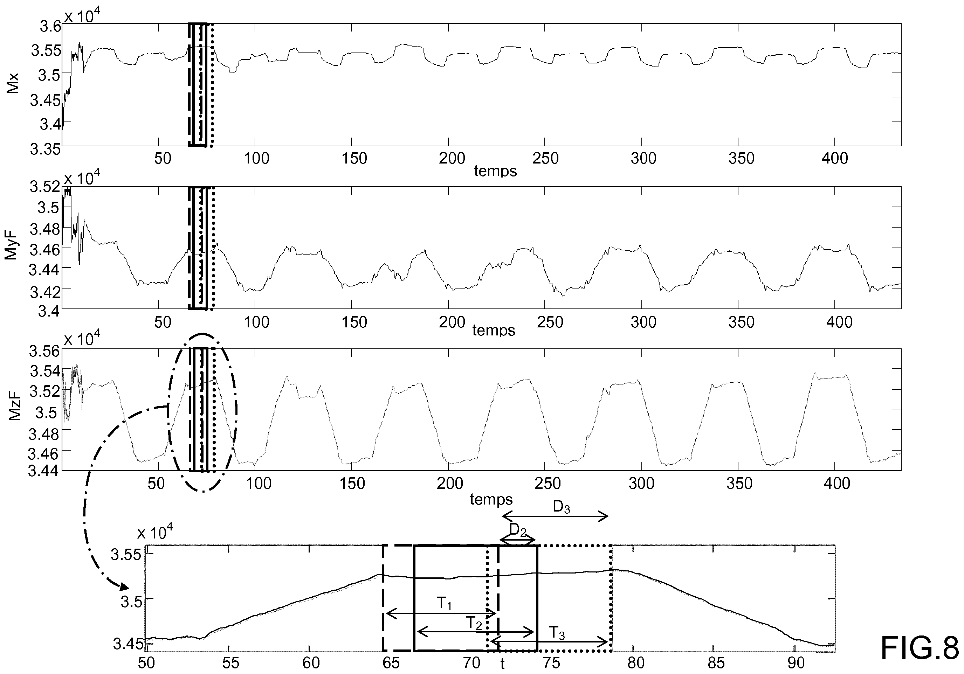

Le module de calcul CALC effectue un fenêtrage, comme illustré sur la figure 8, qui consiste à définir F fenêtres glissantes $\left( F \in \aleph^* \setminus \{1\} \right)$ qui parcourent les composantes du signal de mouvement à analyser. Les F fenêtres, ordonnées temporellement, sont positionnées de manière à respecter la relation suivante :

$$T_1 + D_F \leq S$$

dans laquelle :

T$_1$ représente la durée de la première fenêtre indicée 1,
D$_F$ représente le décalage entre le dernier échantillon de la première fenêtre indicée 1 et le premier échantillon de la dernière fenêtre indicée F, et
S représente toujours la durée minimale de l'activité stationnaire entre deux phases transitoires.

Si la première fenêtre glissante indicée 1, de taille T$_1$ considérée à l'instant t capte le signal filtré MF comme suit :

$$MF1_i^{(t)}(k) = MF_i\left( k + t - T_1 \right), k \in \left[ 1; T_1 \right], i \in \{x, y, z\}$$

Alors les F-1 autres fenêtres glissantes indicées j de taille T$_j$ captent le signal filtré MF aux instants t+D$_j$ en respectant la relation suivante :

$$MFj_i^{(t+D_j)}(k) = MF_i\left( k + t + D_j - T_j \right), k \in \left[ 1; T_j \right], i \in \{x, y, z\}$$

dans laquelle D$_j$ représente le décalage entre le dernier échantillon de la première fenêtre d'indice 1 et la fenêtre d'indice j.

En posant D$_1$=0, il est possible de définir de façon générique les signaux captés par les F fenêtres coulissantes à un instant t donné:

$$MFj_i^{(t+D_j)}(k) = MF_i\left( k + t + D_j - T_j \right), k \in \left[ 1; T_j \right], i \in \{x, y, z\}, \forall j \in \left[ 1; F \right]$$

Avant d'estimer la relation ou le lien statistique entre les signaux issus des F fenêtres glissantes, on se ramène au cas dans lequel les signaux sont tous de même taille.

Pour se ramener au cas de signaux comprenant N échantillons, on peut supprimer (respectivement ajouter) par décimation (respectivement par interpolation) T$_j$-N (respectivement N-T$_j$) échantillons régulièrement espacés (respectivement intercalés) au sein du signal issu de la fenêtre d'indice j et de taille initiale T$_j$>N (respectivement T$_j$<N).

Le calcul du lien statistique consiste à calculer un signal MFC résultant d'une fonction f qui modélise, pour chaque axe de mesure, le lien statistique existant entre les signaux de taille N issus des F fenêtres coulissantes définies à l'instant t comme suit :

$$MFC_i(t) = f\left( MF1_i^{(t)}, ..., MFj_i^{(t+D_j)}, ..., MFF_i^{(t+D_F)} \right), i \in \{x, y, z\}$$

La fonction f qui capte le lien statistique entre les signaux issus des F fenêtres glissantes prend la forme suivante :

$$f\left( MF1_i^{(t)}, ..., MFj_i^{(t+D_j)}, ..., MFF_i^{(t+D_F)} \right) = \sum_{n=1}^{N} \prod_{j=1}^{F} \left( MFj_i^{(t+D_j)}(n) - \mu j_i^{(t+D_j)} \right)^{p_j}$$

*avec*

$$\mu \dot{j}_i^{(t+D_j)} = \frac{1}{N}\sum_{k=1}^{N} MF\dot{j}_i^{(t+D_j)}(k) \quad et \quad p_j \in \aleph$$

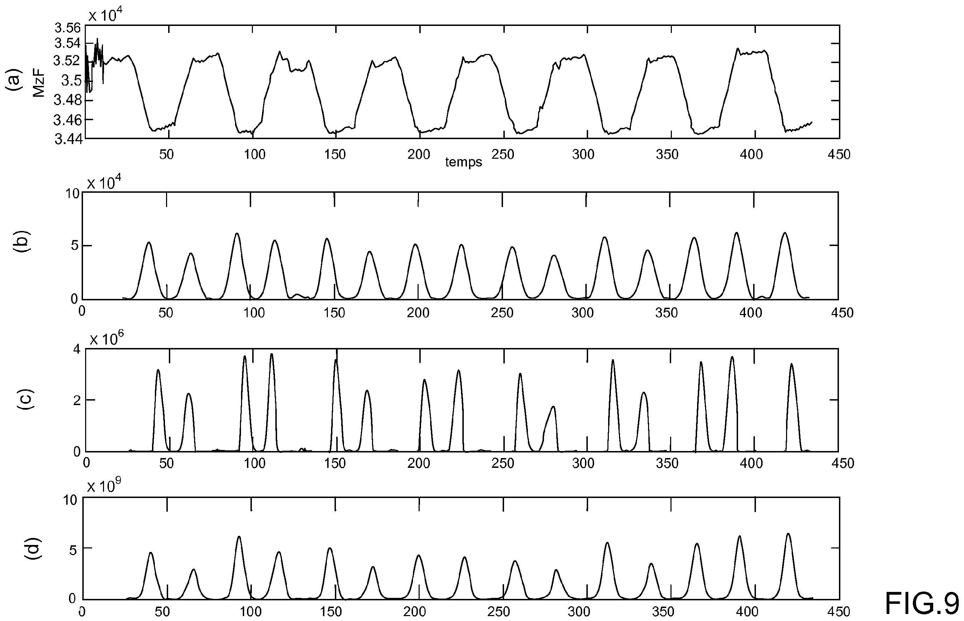

La figure 9 représente quelques exemples de signaux MFC (à une composante) résultant de la fonction f lorsque le nombre de fenêtres glissantes F est égal à deux, pour différents couples de valeurs ($p_1$, $p_2$).

La fonction f capte le lien statistique entre les signaux issus de F=2 fenêtres coulissantes qui parcourent le signal filtré MF à un axe de mesure (a). Le signal résultant est montré pour différentes valeurs de p1 et p2 : p1= p2=1 (b), p1=1 et p2=2 (c), p1= p2=2 (d). Dans tous les cas, les 15 pics indiquent les 15 phases transitoires.

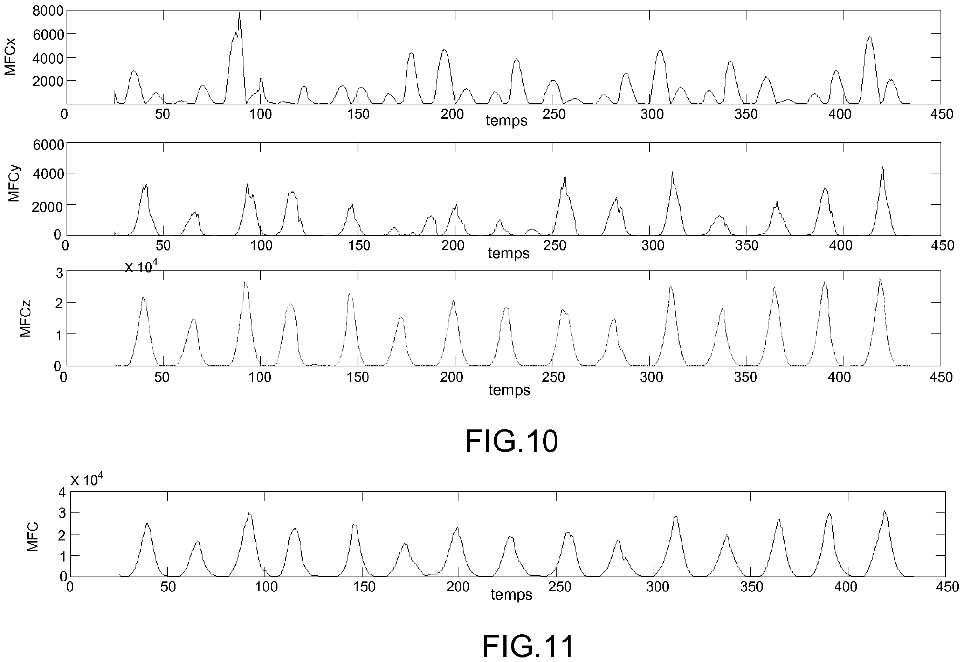

La figure 10 représente un signal MFC à 3 composantes obtenu dans le cas où le nombre de fenêtres glissantes F=2 et pour lesquelles les exposants p1=p2=1. Il est à noter que pour ce cas précis la fonction f correspond à la fonction de covariance.

Le module de fusion FUS permet de condenser l'information en transformant le signal multi-voies ou multi-composantes, en l'occurrence trois composantes, $MFC_i$, $i \in \{x, y, z\}$ en un signal à une seule composante $MFC$ avec

$$MFC(t) = \sum_i MFC_i(t)$$

comme illustré sur la figure 11.

Avant l'étape de détection, il apparait que le signal fusionné présente les caractéristiques voulues pour faciliter la détection des phases transitoires, à savoir : une succession de pics au niveau des demi-tours entrecoupée par des plages de 0 durant l'activité stationnaire (phases de nage ou de sprint).

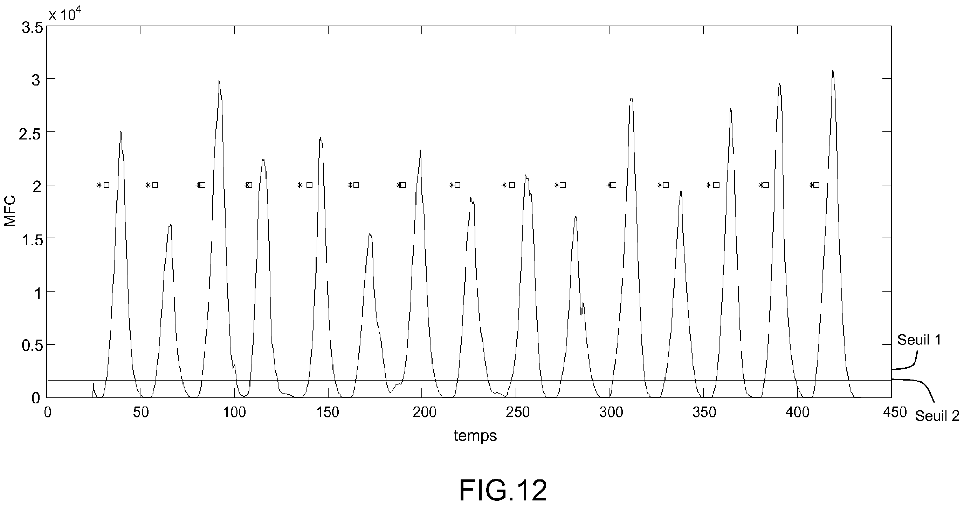

Le module de détection DETEC détecte une première phase transitoire lorsque l'amplitude du signal fusionné MFC devient supérieure à une première valeur de seuil seuil1. Suite à un retournement numéroté j détecté à l'instant T, un retournement numéroté j+1 sera alors détecté à l'instant T+1 à condition que MFC(T+1)>seuil1 et que l'amplitude du signal soit redescendu en-dessous d'une deuxième valeur de seuil seuil2 entre les instants T et T+1. En pratique seuil1>seuil2 comme illustré sur la figure 12. Les valeurs des seuils sont par exemple définies par un apprentissage sur une base de données annotées.

Les étoiles noires sur la figure 12 représentent les instants réels des demi-tours du sprinteur ou du nageur tandis que les carrés indiquent les instants des demi-tours détectés automatiquement par le système. Les traits horizontaux symbolisent respectivement les seuils seuil1 et seuil2.

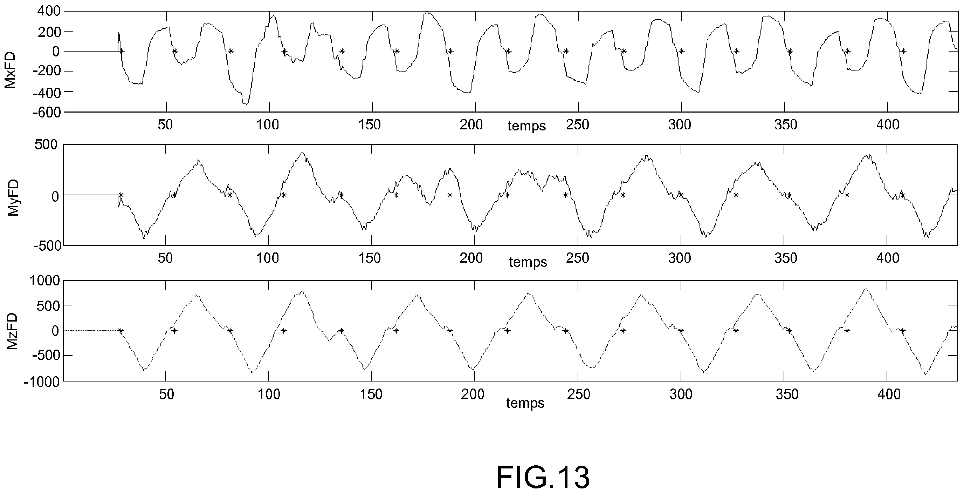

Les performances de détection des phases transitoires par le système peuvent être améliorées en ajoutant une contrainte supplémentaire au moment de la prise de décision, par exemple en faisant intervenir le signe de la composante principale (composante d'énergie la plus élevée) du signal filtré, différencié entre les instants t et t-Δt, comme illustré sur la figure 13 :

$$MFD_i(t) = MF_i(t) - MF_i(t-\Delta t), \Delta t \in \aleph, i \in \{x, y, z\}$$

Δt étant du même ordre de grandeur que la taille L de la fenêtre qui assure le calcul de moyenne.

Sur la figure, le signal à trois composantes filtré et différencié a pour composante maximale, la composante z, qui change de signe à chaque demi-tour.

En plus des conditions sur l'amplitude du signal MFC décrites précédemment, un retournement peut être détecté si le signe de la composante maximale au moment du dépassement du premier seuil seuil1 par MFC est différent du signe de cette même composante lors du retournement précédent. Cette contrainte supplémentaire permet de diminuer le nombre de fausses détections des phases transitoires mais nécessite en contrepartie de différencier le signal MF ce qui sinon n'est pas nécessaire dans la chaîne de traitement proposée. Il est à noter que l'utilisation de ce signe, même si elle améliore les résultats, est totalement facultative et ne modifie en rien les calculs de la chaîne de traitements.

**Revendications**

1. Système de détection d'au moins une phase transitoire dans une activité stationnaire d'un être animé, comprenant des moyens de détermination (DETER) de signaux représentatifs du mouvement dudit être animé selon au moins une voie, **caractérisé en ce qu'**il comprends des moyens de calcul (CALC) d'un signal résultant représentatif d'un lien statistique entre des échantillons desdits signaux représentatifs dudit mouvement appartenant respectivement à au moins deux fenêtres glissantes sur lesdits échantillons, temporellement décalées, et des moyens de détection (DETEC) d'une phase transitoire à partir dudit signal résultant, une activité stationnaire étant définie par une invariance des caractéristiques statistiques desdits échantillons des signaux représentatifs dudit mouvement dans le temps, et une phase transitoire correspondant à une rupture de l'activité stationnaire durant laquelle il y a une modification des caractéristiques statistiques desdits échantillons des signaux représentatifs dudit mouvement.

2. Système selon la revendication 1, comprenant des moyens de filtrage (FILT) desdits signaux représentatifs dudit mouvement en amont desdits moyens de calcul.

3. Système selon l'une des revendications précédentes, dans lequel lesdites fenêtres glissantes se recouvrent partiellement.

4. Système selon l'une des revendications précédentes, dans lequel lesdites fenêtres glissantes sont de même taille.

5. Système selon l'une des revendications précédentes, dans lequel les fenêtres glissantes ordonnées temporellement et respectivement indicées de 1 à F, sont temporellement espacées de manière à respecter la relation suivante :

$$T_1 + D_F \leq S$$

dans laquelle:

$T_1$ représente la durée de la première fenêtre indicée 1,
$D_F$ représente le décalage entre la première fenêtre indicée 1 et la dernière fenêtre indicée F, et
S correspond à une durée minimale de l'activité stationnaire entre deux phases transitoires successives.

6. Système selon l'une des revendications précédentes, dans lequel, lorsque lesdits signaux représentatifs du mouvement sont déterminés selon au moins deux voies de mesure, le système comprend des moyens de fusion (FUS) des composantes selon chaque voie dudit signal résultant.

7. Système selon la revendication 6, dans lequel lesdits moyens de fusion (FUS) sont adaptés pour calculer la somme des composantes selon chaque voie dudit signal résultant.

8. Système selon la revendication 7, dans lequel lesdits moyens de détection (DETEC) sont adaptés pour :

- détecter une première phase transitoire dans l'activité stationnaire lorsque le signal résultant selon une voie unique ou le signal fusionné de sortie des moyens de fusion devient supérieur à un premier seuil (Seuil_1) ; et
- détecter une autre phase transitoire dans l'activité stationnaire lorsque le signal résultant selon une voie unique ou le signal fusionné de sortie des moyens de fusion (FUS), depuis la dernière détection d'une phase transitoire, a été inférieur à un deuxième seuil (Seuil_2) puis est supérieur audit premier seuil (Seuil_1) ;

ledit deuxième seuil (Seuil_2) étant inférieur audit premier seuil (Seuil_1).

9. Système selon la revendication 8 comprenant des moyens de différenciation (DIFF) adaptés pour effectuer une différence entre deux instants desdits signaux représentatifs du mouvement, et des moyens de détermination (DET_CEM) de la composante d'énergie maximale des signaux différenciés, lesdits moyens de détection (DETEC) étant adaptés pour détecter une autre phase transitoire dans l'activité stationnaire lorsqu'en outre le signe de ladite composante maximale, lorsque le signal fusionné est supérieur audit premier seuil (Seuil_1), est l'opposé du signe de ladite composante maximale lors de la détection précédente.

10. Système selon l'une des revendications précédentes, et comprenant des moyens de comptage (CPT) du nombre de phases transitoires.

11. Système selon l'une des revendications précédentes, et comprenant des moyens de chronométrage (CHR) des phases transitoires et/ou stationnaires.

12. Système selon la revendication 10 ou 11, dans lequel lesdits moyens de détection (DETEC) sont adaptés pour détecter des demi-tours d'une personne, entre deux traversées de sens opposés d'une ligne droite, ou pour détecter des changements de direction d'une personne sur un parcours comportant une série de lignes droites.

13. Système selon l'une des revendication précédentes, muni d'un boîtier étanche comprenant lesdits moyens de détermination (DETER) des signaux représentatifs du mouvement d'un nageur et de moyens de fixation pour lier solidairement ledit boîtier à une partie du corps dudit nageur, ladite activité stationnaire étant la nage d'une longueur de piscine et la phase transitoire étant un demi-tour.

14. Système selon la revendication 12, dans lequel lesdits moyens de comptage sont adaptés pour compter les allers-retours d'un nageur dans une piscine.

15. Système selon l'une des revendications précédentes, dans lequel ledit calcul du lien statistique comprend une fonction de la covariance.

16. Procédé de détection d'au moins une phase transitoire dans une activité stationnaire d'un être animé, dans lequel on détermine des signaux représentatifs du mouvement dudit être animé selon au moins une voie, **caractérisé en ce qu'**on calcule un signal résultant représentatif d'un lien statistique entre des échantillons desdits signaux représentatifs dudit mouvement appartenant respectivement à au moins deux fenêtres glissantes sur lesdits échantillons, temporellement décalées, une activité stationnaire étant définie par une invariance des caractéristiques statistiques de l'activité dans le temps, et une phase transitoire correspondant à une rupture de l'activité stationnaire durant laquelle il y a une modification des caractéristiques statistiques.

**Patentansprüche**

1. System zum Erkennen von wenigstens einer Übergangsphase in einer stationären Aktivität eines Lebewesens, das Mittel zum Bestimmen (DETER) von für die Bewegung des Lebewesens über wenigstens einen Weg repräsentativen Signalen umfasst, **dadurch gekennzeichnet, dass** es Mittel (CALC) zum Berechnen eines resultierenden Signals, das für eine statistische Verbindung zwischen Proben der für die Bewegung repräsentativen Signale repräsentativ ist, die jeweils zu wenigstens zwei zeitlich versetzten Gleitfenstern auf den Proben gehören, und Mittel (DETEC) zum Erkennen einer Übergangsphase auf der Basis des resultierenden Signals umfasst, wobei eine stationäre Aktivität durch eine Invarianz der statistischen Charakteristiken der Proben der für die Bewegung über die Zeit repräsentativen Signale definiert wird, und einer Übergangsphase, die einer Unterbrechung der stationären Aktivität entspricht, bei der eine Änderung der statistischen Charakteristiken der Proben der für die Bewegung repräsentativen Signale stattfindet.

2. System nach Anspruch 1, das Mittel zum Filtern (FILT) der für die Bewegung repräsentativen Signale stromaufwärts von den Rechenmitteln umfasst.

3. System nach einem der vorherigen Ansprüche, bei dem die Gleitfenster teilweise überlappen.

4. System nach einem der vorherigen Ansprüche, bei dem die Gleitfenster dieselbe Größe haben.

5. System nach einem der vorherigen Ansprüche, bei dem die zeitlich geordneten Gleitfenster jeweils mit 1 bis F indexiert und zeitlich beanstandet sind, um die folgende Beziehung einzuhalten:

$$T_1 + D_F \leq S$$

wobei:

$T_1$ die Dauer des ersten Fensters mit Index 1 repräsentiert,
$D_F$ den Versatz zwischen dem ersten Fenster mit Index 1 und dem letzten Fenster mit Index F repräsentiert, und
S einer minimalen Dauer der stationären Aktivität zwischen zwei aufeinanderfolgenden Übergangsphasen entspricht.

6. System nach einem der vorherigen Ansprüche, bei dem beim Bestimmen der für die Bewegung repräsentativen Signale gemäß zwei Messwegen das System Fusionsmittel (FUS) der Komponenten gemäß jedem Weg des resultierenden Signals umfasst.

7. System nach Anspruch 6, bei dem die Fusionsmittel (FUS) zum Berechnen der Summe der Komponenten auf jedem Weg des resultierenden Signals ausgelegt sind.

8. System nach Anspruch 7, bei dem die Erkennungsmittel (DETEC) ausgelegt sind zum:

- Erkennen einer ersten Übergangsphase in der stationären Aktivität, wenn das von einem einzigen Weg resultierende Signal oder das fusionierte Ausgangssignal der Fusionsmittel größer wird als eine erste Schwelle (seuil_1); und
- Erkennen einer anderen Übergangsphase in der stationären Aktivität, wenn das von einem einzigen Weg resultierende Signal oder das fusionierte Ausgangssignal von Fusionsmitteln (FUS) seit der letzten Erkennung einer Übergangsphase kleiner geworden ist als eine zweite Schwelle (Seuil_2) und dann größer geworden ist als die erste Schwelle (seuil_1);

wobei die zweite Schwelle (Seuil_2) kleiner ist als die erste Schwelle (Seuil_1).

9. System nach Anspruch 8, das Differenzierungsmittel (DIFF) zum Bewirken einer Differenz zwischen zwei Instanzen der für die Bewegung repräsentativen Signale und Mittel (DET_CEM) zum Bestimmen der maximalen Energiekomponente der differenzierten Signale umfasst, wobei die Erkennungsmittel (DETEC) zum Erkennen einer anderen Übergangsphase in der stationären Aktivität ausgelegt sind, wenn ferner das Signal der maximalen Komponente, wenn das fusionierte Signal größer ist als die erste Schwelle (Seuil_1), das entgegengesetzte Vorzeichen zu der maximalen Komponente während der vorhergehenden Erkennung hat.

10. System nach einem der vorherigen Ansprüche, das Mittel (CPT) zum Zählen der Anzahl von Übergangsphasen umfasst.

11. System nach einem der vorherigen Ansprüche, das Mittel (CHR) zum Messen der Zeit der Übergangsphasen und/oder der stationären Phasen umfasst.

12. System nach Anspruch 10 oder 11, bei dem die Erkennungsmittel (DETEC) zum Erkennen von Wenden einer Person zwischen zwei Überquerungen einer Geraden im entgegengesetzten Sinne oder zum Erkennen von Richtungswechseln einer Person auf einer Strecke ausgelegt sind, die eine Serie von Geraden umfasst.

13. System nach einem der vorherigen Ansprüche, ausgestattet mit einem dichten Gehäuse, das die Mittel (DETER) zum Bestimmen der für die Bewegung eines Schwimmers repräsentativen Signale und Befestigungsmittel zum festen Verbinden des Gehäuses mit einem Teil des Körpers des Schwimmers umfasst, wobei die stationäre Aktivität das Durchschwimmen einer Schwimmbeckenlänge ist und die Übergangsphase eine Wende ist.

14. System nach Anspruch 12, bei dem die Zählmittel zum Zählen der Hin- und Rückbahnen eines Schwimmers in einem Schwimmbecken ausgelegt sind.

15. System nach einem der vorherigen Ansprüche, bei dem das Berechnen der statistischen Verbindung eine Kovarianzfunktion umfasst.

16. Verfahren zum Erkennen von wenigstens einer Übergangsphase in einer stationären Aktivität eines Lebewesens, in dem die für die Bewegung des Lebewesens über wenigstens einen Weg repräsentativen Signale bestimmt werden, **dadurch gekennzeichnet, dass** ein resultierendes Signal berechnet wird, das für eine statistische Verbindung zwischen den Proben der für die Bewegung repräsentativen Signale, die jeweils zu wenigstens zwei zeitlich versetzten Gleitfenstern der Proben gehören, repräsentativ sind, wobei eine stationäre Aktivität durch eine Invarianz der statistischen Charakteristiken der Aktivität über die Zeit definiert wird, und wobei eine Übergangsphase einer Unterbrechung der stationären Aktivität entspricht, während der eine Modifikation der statistischen Charakteristiken stattfindet.

**Claims**

1. System for detecting at least one transient phase in a stationary activity of an animate being, comprising determining means (DETER) for signals representative of the movement of the animate being along at least one path, **characterised in that** it comprises calculating means (CALC) for a resulting signal which is representative of a statistical link between the samples of the signals representative of the movement which belong respectively to at least two sliding windows on the samples, which are temporally offset, and detecting means (DETECT) for a transient phase from the resulting signal, a stationary activity being defined by an invariance of the statistical characteristics of the samples of the signals representative of the movement over time and a transient phase which corresponds to an interruption of the stationary activity during which there is a modification of the statistical characteristics of the samples of the signals representative of the movement.

2. System according to claim 1, comprising filtering means (FILT) for the signals representative of the movement upstream of the calculating means.

3. System according to either of the preceding claims, wherein the sliding windows partially overlap.

4. System according to any one of the preceding claims, wherein the sliding windows are of the same size.

5. System according to any one of the preceding claims, wherein the sliding windows which are temporally ordered and indexed 1 to F, respectively, are temporally spaced apart in order to comply with the following relationship:

$$T_1 + D_F \leq S$$

wherein

$T_1$ represents the duration of the first window indexed 1,
$D_F$ represents the offset between the first window indexed 1 and the final window indexed F, and
S corresponds to a minimum duration of the stationary activity between two successive transient phases.

6. System according to any one of the preceding claims, wherein, when the signals representative of the movement are determined in accordance with at least two measurement paths, the system comprises merging means (FUS) for the components along each path of the resulting signal.

7. System according to claim 6, wherein the merging means (FUS) are adapted to calculate the total of the components along each path of the resulting signal.

8. System according to claim 7, wherein the detecting means (DETEC) are adapted to:

- detect a first transient phase in the stationary activity when the resulting signal along a single path or the merged output signal of the merging means becomes greater than a first threshold (seuil_1); and
- detect another transient phase in the stationary activity when the resulting signal along a single path or the merged output signal of the merging means (FUS), since the last detection of a transient phase, was lower than a second threshold (Seuil_2) then is greater than the first threshold (seuil_1);

the second threshold (Seuil_2) being lower than the first threshold (Seuil_1).

9. System according to claim 8 comprising differentiation means (DIFF) which are adapted to carry out a differentiation between two times of the signals representative of the movement, and determining means (DET_CEM) for the maximum energy component of the differentiated signals, the detecting means (DETEC) being adapted to detect another transient phase in the stationary activity when furthermore the sign of the maximum component when the merged signal is greater than the first threshold (Seuil_1), is the opposite of the sign of the maximum component during the previous detection.

10. System according to any one of the preceding claims, and comprising counting means (CPT) for the number of transient phases.

11. System according to any one of the preceding claims, and comprising timing means (CHR) for the transient and/or stationary phases.

12. System according to claim 10 or 11, wherein the detecting means (DETEC) are adapted to detect half-turns of a person, between two crossings in opposite directions of a straight line, or to detect changes of direction of a person over a path which comprises a series of straight lines.

13. System according to any one of the preceding claims, provided with a sealed casing which comprises the determining means (DETER) for the signals representative of the movement of a swimmer and fixing means to fixedly join the casing to a portion of the body of the swimmer, the stationary activity being the swimming of one length of a swimming pool and the transient phase being a half-turn.

14. System according to claim 12, wherein the counting means are adapted to count the round trips of a swimmer in a swimming pool.

15. System according to any one of the preceding claims, wherein the calculation of the statistical link comprises a function of the covariance.

16. Method for detecting at least one transient phase in a stationary activity of an animate being, wherein there are determined signals which are representative of the movement of the animate being along at least one path, **characterised in that** there is calculated a resulting signal which is representative of a statistical link between samples of the signals representative of the movement which belong respectively to at least two sliding windows on the samples, which are temporally offset, a stationary activity being defined by a non-variance of the statistical characteristics of the activity over time, and a transient phase which corresponds to an interruption of the stationary activity during which there is a modification of the statistical characteristics.

```
┌───────┐  Signaux      ┌──────┐  Signal lien   ┌───────┐
│ DETER │ ───────────→  │ CALC │ ────────────→  │ DETEC │        FIG.1
└───────┘  mouvements   └──────┘  statistique   └───────┘
```

```
┌───────┐  Signaux    ┌──────┐   ┌──────┐  Signal lien   ┌───────┐
│ DETER │ ─────────→  │ FILT │ → │ CALC │ ────────────→  │ DETEC │   FIG.2
└───────┘  mouvements └──────┘   └──────┘  statistique   └───────┘
```

```
┌───────┐  Signaux    ┌──────┐   ┌──────┐  Signal lien  ┌─────┐   ┌───────┐
│ DETER │ ─────────→  │ FILT │ → │ CALC │ ───────────→  │ FUS │ → │ DETEC │   FIG.3
└───────┘  mouvements └──────┘   └──────┘  statistique  └─────┘   └───────┘
```

```
┌───────┐  Signaux    ┌──────┐   ┌──────┐  Signal lien  ┌─────┐   ┌───────┐   ┌─────┐
│ DETER │ ─────────→  │ FILT │ → │ CALC │ ───────────→  │ FUS │ → │ DETEC │ → │ CPT │   FIG.4
└───────┘  mouvements └──────┘   └──────┘  statistique  └─────┘   └───────┘   └─────┘
```

```
┌───────┐  Signaux    ┌──────┐   ┌──────┐  Signal lien  ┌─────┐   ┌───────┐   ┌─────┐
│ DETER │ ─────────→  │ FILT │ → │ CALC │ ───────────→  │ FUS │ → │ DETEC │ → │ CPT │
└───────┘  mouvements └──────┘   └──────┘  statistique  └─────┘   └───────┘   └─────┘
                           │                                          ↑
                           ↓                                          │          FIG.5
                      ┌──────┐        ┌─────────┐                     │
                      │ DIFF │ ────→  │ DET_CEM │ ────────────────────┘
                      └──────┘        └─────────┘
```

FIG.6

EP 2 793 700 B1

FIG.7

EP 2 793 700 B1

FIG.8

FIG.9

FIG.10

FIG.11

FIG.12

FIG.13

EP 2 793 700 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4932045 A
- US 20070293374 A1

- WO 2011012666 A